(19) Europäisches Patentamt / European Patent Office / Office européen des brevets

(11) **EP 3 192 530 A1**

(12) **DEMANDE DE BREVET EUROPEEN**

(43) Date de publication:
19.07.2017 Bulletin 2017/29

(51) Int Cl.:
*A61K 49/12* (2006.01)          *A61K 49/18* (2006.01)
*A61K 51/06* (2006.01)          *A61K 51/12* (2006.01)
*B82Y 15/00* (2011.01)

(21) Numéro de dépôt: 16305038.8

(22) Date de dépôt: 15.01.2016

(84) Etats contractants désignés:
AL AT BE BG CH CY CZ DE DK EE ES FI FR GB GR HR HU IE IS IT LI LT LU LV MC MK MT NL NO PL PT RO RS SE SI SK SM TR
Etats d'extension désignés:
BA ME
Etats de validation désignés:
MA MD

(71) Demandeurs:
• **INSTITUT NATIONAL DE LA SANTE ET DE LA RECHERCHE MEDICALE (INSERM)**
  **75013 Paris (FR)**
• **Université Claude Bernard Lyon I**
  **69100 Villeurbanne (FR)**
• **Université de Paris 5 René Descartes**
  **75006 Paris (FR)**
• **Centre National de la Recherche Scientifique**
  **75016 Paris (FR)**
• **Ecole Nationale Superieure de Chimie de Paris**
  **75005 Paris (FR)**

(72) Inventeurs:
• MIGNET, Nathalie
  92140 CLAMART (FR)
• DOAN, Bich-Thuy
  78420 CARRIÈRES SUR SEINE (FR)
• RAMNICEANU, Grégory
  75006 PARIS (FR)
• TILLEMENT, Olivier
  69270 FONTAINES SAINT MARTIN (FR)
• SANCEY GALLIOT, Lucie
  38850 PALADRU (FR)
• LUX, François
  69003 LYON (FR)
• SEGUIN, Johanne
  94270 KREMLIN BICÊTRE (FR)

(74) Mandataire: Marro, Nicolas
  Cabinet Beau de Lomenie
  158 rue de l'Université
  75340 Paris Cedex 07 (FR)

(54) **NANOPARTICULES ULTRAFINES COMME AGENT D'IMAGERIE POUR EL DIAGNOSTIC D'UNE DYSFONCTION RÉNALE**

(57) L'invention concerne une nouvelle utilisation de nanoparticules ultrafines comme agent d'imagerie dans une méthode de diagnostic d'une dysfonction rénale. L'invention vise également l'utilisation de nanoparticules ultrafines comme agent d'imagerie dans des méthodes de suivi de l'efficacité thérapeutique d'un traitement d'une dysfonction rénale. Lesdites nanoparticules comprennent (i) une matrice de polyorganosiloxane (POS), (ii) un ou plusieurs agents chélatants greffés à la matrice POS par liaison covalente -Si-C- et (iii) un ou plusieurs ions métalliques chélatés par un ou plusieurs des agents chélatants susmentionnés.

FIGURE 1

EP 3 192 530 A1

## Description

Domaine technique

L'invention concerne une nouvelle utilisation de nanoparticules ultrafines, comme agent de diagnostic d'une dysfonction rénale.

Arrière-plan technologique

Le rein est un organe qui a pour fonction d'épurer le sang. Il peut ainsi filtrer jusqu'à 170 litres de sang par jour chez l'Homme. En filtrant le sang, le rein produit l'urine. L'urine est un mélange composé d'eau, de sels minéraux (sodium, potassium, calcium...) et de déchets toxiques comme l'urée et la créatinine.

Outre la filtration du sang et la production d'urine, le rein remplit également d'autres fonctions physiologiques importantes comme la régulation de la teneur en eau et en sel du corps, la production d'érythropoïétine, indispensable à la formation des globules rouges, la production de la forme active de vitamine D qui intervient dans le maintien de la teneur en calcium sanguin, la régulation de la composition des liquides corporels, ou la production de rénine intervenant dans la régulation de la pression artérielle.

Les pathologies rénales peuvent être de différents types et se traduisent par une dysfonction rénale. La dysfonction rénale se traduit généralement par une diminution de la fonctionnalité rénale, en particulier une diminution de la fonction tubulaire et/ou une diminution de la filtration glomérulaire. Elle peut aller jusqu'à une insuffisance rénale dans les cas les plus sévères.

L'insuffisance rénale correspond à l'altération du fonctionnement des reins qui ne filtrent plus correctement le sang. La maladie est dite aiguë si le dysfonctionnement est transitoire et réversible et chronique si la destruction est irréversible, sans possibilité de guérison. Si l'insuffisance rénale est majeure, la fonction rénale peut être supplantée par dialyse ou transplantation. La dialyse permet de filtrer le sang par un circuit dérivé, le plus souvent extérieur à l'organisme.

L'insuffisance rénale aiguë survient le plus souvent après une agression comme une baisse brutale et transitoire de la pression artérielle, lors d'une hémorragie, d'une infection générale (septicémie), d'une intoxication médicamenteuse ou encore en cas d'obstruction des voies urinaires par un calcul ou un adénome prostatique. Les reins mettent quelques jours à retrouver spontanément un fonctionnement normal après traitement. Pendant cette période, il est parfois recommandé de recourir à la dialyse qui permet au patient de survivre pendant le processus d'autoréparation rénale.

L'insuffisance rénale chronique ne régresse pas, par définition. Elle peut être induite par des pathologies (diabète, hypertension...) qui détruisent progressivement et de façon irréversible les différentes structures rénales. Il existe cinq stades de la maladie jusqu'au stade terminal au cours duquel la capacité de filtration est inférieure à 15 % de la normale pour l'ensemble des reins. Ce stade nécessite d'envisager les techniques de remplacement de la fonction rénale : dialyse et/ou transplantation.

Les personnes touchées par une insuffisance rénale peuvent rester en bonne santé apparente avec des reins fonctionnant de 10 à 20% de leur capacité normale. En effet, les principaux symptômes de l'insuffisance rénale ne se déclarent qu'à un stade avancé, ce qui rend difficile le diagnostic de la maladie. Il est néanmoins important d'agir tôt pour éviter les complications liées à l'insuffisance rénale et/ou empêcher l'évolution de l'insuffisance rénale.

Selon une étude menée aux Etats-Unis sur la période 1999-2000 (*National Health and Nutrition Examination Survey IV*), 9,4 % de la population générale, dont deux tiers d'hommes, présenteraient une insuffisance rénale, dont 5,6 % à un stade léger ou modéré, 3,7 % à un stade sévère et 0,13 % à un stade terminal (Coresh J, Byrd-Holt D, Astor BC, et al. Chronic kidney disease awareness, prevalence, and trends among U.S. adults, 1999 to 2000. JAm Soc Nephrol 2005;16:180-8). La maladie est rare avant 45 ans mais sa prévalence augmente avec l'âge, notamment après 65 ans. La prévalence de cette maladie devrait encore augmenter dans les années qui viennent en raison du vieillissement de la population et de l'augmentation du diabète, deux causes majeures d'insuffisance rénale, et grâce à l'amélioration de la survie des patients transplantés et dialysés.

L'insuffisance rénale peut être la conséquence d'une fibrose rénale. La fibrose rénale est due à une accumulation excessive de matrice extracellulaire dans le parenchyme rénal. Elle résulte d'une déstabilisation d'un équilibre complexe entre cellules et protéines profibrosantes et antifibrosantes. Les mécanismes précis régulant l'apparition de la fibrose ne sont qu'incomplètement élucidés à ce jour et il n'existe pas, pour l'instant, de traitement efficace de la fibrose permettant le retour à un parenchyme rénal normalement fonctionnel une fois que la maladie est installée. Ainsi, la fibrose rénale conduit plus ou moins rapidement vers une insuffisance rénale chronique terminale.

Il est donc essentiel de pouvoir diagnostiquer efficacement la dysfonction rénale afin de prendre en charge au plus vite les patients et ainsi empêcher la progression de la maladie, notamment la progression vers une insuffisance rénale chronique.

Généralement, la dysfonction rénale est diagnostiquée par une analyse des urines et/ou du sang. Les marqueurs à prendre en compte peuvent être de différentes natures. Par exemple, la Haute Autorité de Santé (HAS) préconise d'évaluer la fonction rénale à partir de la créatinémie, par l'estimation du débit de filtration glomérulaire (DFG) et le dosage de la créatinine par une méthode enzymatique.

Néanmoins, le dosage de la créatinine n'est pas suffisant car il n'y a pas de relation linéaire entre la créatinémie et la filtration glomérulaire (filtration rénale). De plus, les analyses sanguines et/ou urinaires ne permettent pas toujours de poser un diagnostic précis de la dysfonction rénale, notamment de l'insuffisance rénale. En effet, ces analyses ne sont que des éléments qui permettent d'orienter le médecin et peuvent être révélatrices d'autres pathologies. Afin de confirmer la dysfonction rénale, il est souvent nécessaire d'effectuer d'autres analyses, voire de prendre l'avis de spécialistes comme un néphrologue, un urologue ou un cardiologue. Le diagnostic peut alors être long et couteux, puisqu'il nécessite la réalisation de plusieurs analyses et la consultation de différents spécialistes.

Par ailleurs, pour certains types de patients, il peut être recommandé de réaliser d'autres analyses comme par exemple une recherche de microalbuminurie chez les patients diabétiques de type 1 et 2.

Les techniques d'imagerie peuvent également être utilisées pour diagnostiquer la dysfonction rénale, comme par exemple la radiologie, l'échographie, la tomodensitométrie (TDM) ou l'imagerie par résonnance magnétique (IRM). Ces techniques sont généralement utilisées pour identifier les causes de la dysfonction rénale.

Il existe donc un réel besoin de diagnostiquer de manière précise et fiable tout type de dysfonction rénale.

Les inventeurs ont montré que des nanoparticules ultrafines, d'un diamètre hydrodynamique moyen inférieur à 10 nm, avantageusement inférieur à 5 nm, par exemple allant de 1 à 5 nm, peuvent être utilisées comme agent d'imagerie particulièrement favorable pour l'imagerie du rein. Ces nanoparticules permettent d'obtenir des informations fiables et précises sur la fonction rénale, notamment par imagerie IRM. Ainsi, ces nanoparticules ultrafines sont particulièrement avantageuses pour être utilisées dans une méthode de diagnostic par imagerie d'une dysfonction rénale, telle que la fibrose rénale et/ou l'insuffisance rénale.

Résumé de l'invention

La présente invention vise à proposer de nouvelles méthodes d'imagerie, notamment par IRM, permettant la détection fiable d'une dysfonction rénale, notamment la fibrose rénale et/ou l'insuffisance rénale, éventuellement couplées à un acte thérapeutique.

Selon un premier aspect, l'invention concerne des nanoparticules destinées à être utilisées comme agent d'imagerie dans une méthode de diagnostic *in vivo* d'une dysfonction rénale chez l'homme ou l'animal, lesdites nanoparticules comprenant :

(i) une matrice de polyorganosiloxane (POS) ;
(ii) un ou plusieurs agents chélatants, de préférence de 6 à 20 agents chélatants, greffés à la matrice POS par liaison covalente -Si-C- ;
(iii) un ou plusieurs ions métalliques chélatés par un ou plusieurs des agents chélatants susmentionnés.

Selon un deuxième aspect, l'invention concerne l'utilisation des nanoparticules telles que définies ci-dessus, comme agent d'imagerie dans le diagnostic *in vivo* d'une dysfonction rénale chez l'homme ou l'animal.

Selon un troisième aspect, l'invention concerne une méthode pour diagnostiquer une dysfonction rénale *in vivo* chez l'homme ou l'animal auquel ont été administrées des nanoparticules telles que définies ci-dessus.

Selon un quatrième aspect, l'invention concerne une méthode de suivi de l'efficacité thérapeutique du traitement d'une dysfonction rénale chez l'homme ou l'animal auquel ont été administrées des nanoparticules ultrafines selon l'invention, de préférence par voie intraveineuse, ladite méthode comprenant les étapes suivantes :

(i) on capture des images par une technique d'imagerie appropriée afin de visualiser au niveau du rein desdites nanoparticules ;
(ii) on détermine le rehaussement ;
(iii) on répète les étapes (i) et (ii) au cours du traitement du sujet, autant que nécessaire ;
(iv) on déduit l'efficacité thérapeutique du traitement en comparant l'évolution du rehaussement au cours du traitement.

Selon un cinquième aspect, l'invention concerne une méthode de suivi de l'efficacité thérapeutique d'un traitement d'une dysfonction rénale chez l'homme ou l'animal, ladite méthode comprenant les étapes suivantes :

(i) à l'initiation du traitement, on administre au patient, des nanoparticules ultrafines selon l'invention, à titre d'agent d'imagerie,
(ii) on capture des images par une technique d'imagerie appropriée afin de visualiser au niveau du rein lesdites nanoparticules
(iii) on détermine le rehaussement ;
(iv) on répète les étapes (i) et (iii) au cours du traitement du sujet, autant que nécessaire ;
(v) on déduit l'efficacité thérapeutique du traitement en comparant l'évolution du rehaussement au cours du traitement.

Description détaillée de l'invention

La présente invention découle des avantages surprenants mis en évidence par les inventeurs d'une administration de certaines nanoparticules ultrafines, utilisables comme agent d'imagerie, notamment en IRM $T_1$, pour le diagnostic d'une dysfonction rénale, notamment la fibrose rénale. L'ensemble des applications qui en découlent sont ainsi liées aux caractéristiques chimiques et structurales de ces nanoparticules qui sont décrites ci-après.

L'invention concerne en effet, des nanoparticules destinées à être utilisées comme agent d'imagerie dans une méthode de diagnostic *in vivo* d'une dysfonction rénale chez l'homme ou l'animal, lesdites nanoparticules comprenant :

(i) une matrice de polyorganosiloxane (POS) ;
(ii) un ou plusieurs agents chélatants, de préférence de 6 à 20 agents chélatants, greffés à la matrice POS par liaison covalente -Si-C- ;
(iii) un ou plusieurs ions métalliques chélatés par un ou plusieurs des agents chélatants susmentionnés.

Au sens de l'invention, le terme « diagnostic » et « diagnostic par imagerie » peuvent être utilisés indifféremment.

Au sens de l'invention, on entend par « agent d'imagerie » tout produit ou composition utilisé en imagerie médicale dans le but d'augmenter artificiellement le contraste permettant de visualiser une structure anatomique particulière (par exemple certains tissus ou un organe).

Dans un mode de réalisation particulier, les nanoparticules ultrafines selon l'invention sont utilisées comme agent d'imagerie pour les applications en imagerie par résonnance magnétique (IRM), notamment en IRM dynamique (i.e. DCE pour Dynamic Contrast Enhanced sequence). Dans ce mode de réalisation, les nanoparticules comprennent au moins un ion métallique pour l'imagerie IRM $T_1$, particulièrement appropriée pour l'imagerie des reins. L'IRM permet notamment d'obtenir une précision spatio-temporelle particulièrement avantageuse pour la mise en oeuvre de la présente invention.

Au sens de l'invention, on entend par « ion métallique » tout ion métallique qui permet de conférer à l'agent d'imagerie ses propriétés d'augmentation du contraste, c'est-à-dire ses propriétés d'augmentation du signal. Le choix de de l'ion métallique sera déterminé en fonction de la technique d'imagerie utilisé.

Dans un mode de réalisation particulier, l'ion métallique est un ion d'un métal radioactif, tel qu'un cation d'un ion métallique radioactif $M^{n+}$, n étant un nombre entier allant de 2 à 4, par exemple égal à 2, égal à 3 ou égal à 4. En particulier, le cation d'un ion métallique radioactif est un lanthanide, de préférence choisi parmi Dy (Dysprosium), Lu (Lutecium), Gd (Gadolinium), Ho (Holmium), Eu (Europium), Tb (Terbium), Nd (Néodymium), Er (Erbium), Yb (Ytterbium) ou leurs mélanges, et plus préférentiellement encore Gd. Dans un mode de réalisation particulier, le métal radioactif est le Fer (Fe) ou le Manganèse (Mn).

De préférence, on choisira à titre d'ion métallique au moins un cation de lanthanide, et incluant au moins 50% de Gd, Dy ou Ho ou leurs mélanges, de préférence le Gd, par exemple au moins 50% de Gd, par exemple 100% de Gd (c'est-à-dire uniquement du $Gd^{3+}$ comme ion métallique). En particulier, le $Gd^{3+}$ permet d'obtenir un signal optimal en modalité d'imagerie, notamment en modalité d'imagerie IRM.

Les nanoparticules selon l'invention peuvent être utilisées dans tout type d'imagerie, notamment l'une des techniques d'imageries suivantes :

(i) l'IRM, de préférence l'IRM $T_1$,
(ii) la scintigraphie TEP (Tomographie par Emission de Positons) ou TEMP (Tomographie par Emission Monophotonique),
(iii) la fluorescence dans le visible ou dans le proche infra-rouge,
(iv) la tomodensitométrie aux rayons X.

Pour l'imagerie en scintigraphie, les nanoparticules comprennent un isotope radioactif susceptible d'être utilisé en scintigraphie, et de préférence choisi dans le groupe constitué par des isotopes radioactifs de In, Te, Ga, Cu, Zr, Y ou Lu, par exemple $^{111}$In, $^{99m}$Tc, $^{68}$Ga, $^{64}$Cu, $^{89}$Zr, $^{90}$Y, $^{177}$Lu. L'isotope radioactif est alors chélaté par un agent chélatant greffé à la matrice POS.

Pour la fluorescence dans le visible on peut utiliser un lanthanide choisi parmi Eu ou Tb. Le lanthanide est alors chélaté par un agent chélatant greffé à la matrice POS.

Pour la fluorescence dans le proche infra-rouge, on pourra utiliser par exemple un fluorophore approprié, par exemple un fluorophore organique comme une cyanine, une alexa, une bodipy, un indocyanine green (ICG). Le fluorophore est avantageusement greffé sur la nanoparticule, avantageusement par greffage covalent en utilisant une méthode de greffage appropriée.

Pour la tomodensitométrie aux rayons X, les mêmes ions métalliques que pour l'IRM peuvent être utilisés. On pourra utiliser par exemple un lanthanide, tel que le Gd.

Dans un mode de réalisation particulier, les nanoparticules utilisables selon l'invention sont caractérisées en ce qu'elles comprennent au moins un ion métallique pour l'imagerie IRM $T_1$, et éventuellement au moins un autre ion métallique approprié pour l'une des techniques d'imageries suivantes :

(i) la scintigraphie TEP (Tomographie par Emission de Positons) ou TEMP (Tomographie par Emission Monophotonique),
(ii) la fluorescence dans le visible ou dans le proche infra-rouge,
(iii) la tomodensitométrie aux rayons X.

Selon l'invention, un ou plusieurs ions métalliques sont chélatés par un ou plusieurs des agents chélatants greffés aux nanoparticules.

Au sens de l'invention, on entend par « agent chélatant » un corps chimique agissant comme un chélate, c'est-à-dire un corps chimique qui a la propriété de fixer durablement des ions positifs.

Les nanoparticules de l'invention comprennent un ou plusieurs agents chélatants, de préférence de 6 à 20, avantageusement de 8 à 10 agents chélatants, greffés à la matrice POS par liaison covalente -Si-C.

Selon l'invention, une partie ou la totalité de ces agents chélatants sont destinés à complexer des ions métalliques, de préférence des cations $M^{n+}$ (e.g. du gadolinium). Une autre partie de ces agents chélatants peut servir à la complexation de cations endogènes pour assurer une bonne compatibilité avec les milieux biologiques rencontrés. Dans un mode de réalisation préféré, tous les agents chélatants sont chélatés avec un ion métallique, de préférence des cations $M^{n+}$ (e.g. du $Gd^{3+}$).

Avantageusement, l'agent chélatant est choisi parmi les produits suivants :

- les produits du groupe des acides polycarboxyliques polyaminés et leurs dérivés, et, plus préférentiellement encore dans le sous-groupe comprenant : DOTA (acide 1,4,7,10-tétraazacyclododécane-1,4,7,10-tétraacétique), DTPA (acide diéthylène triamine pentaacétique), EDTA (acide éthylène diamine tétraacétique), EGTA (acide éthylene-bis-(oxyéthylènenitrilo)tétraacétique), BAPTA (acide 1,2-bis o-aminophénoxyéthane-N,N,N',N'-tétraacétique), NOTA (acide 1,4,7-triazacyclononane-1,4,7 triacétique) et leurs mélanges ;
- les produits du groupe comprenant porphyrine, chlorine, 1,10-phénanthroline, bipyridine, terpyridine, cyclam, triazacyclononane, leurs dérivés et leurs mélanges;
- et leurs mélanges.

Si l'ion métallique est un lanthanide, l'agent chélatant est avantageusement sélectionné parmi ceux qui présentent des propriétés complexantes des lanthanides, en particulier ceux dont la constante de complexation $\log(K_{Ci})$ est supérieure à 15, préférentiellement 20. Comme exemples préférés d'agents chélatants, complexant des lanthanides, on peut citer ceux comprenant un motif d'acide diéthylène triamine penta acétique (DTPA), l'acide 1,4,7,10-tétraazacyclododécane-1,4,7,10-tétraacétique (DOTA), l'acide 1,4,7-triazacyclononane-1,4,7-triacétique (NOTA) ou leurs dérivés.

Dans un mode de réalisation préféré, l'ion métallique est $Gd^{3+}$ et l'agent chélatant est l'acide 1,4,7,10-tétraazacyclododécane-1,4,7,10-tétraacétique (DOTA). Cette combinaison est particulièrement avantageuse en IRM $T_1$.

Avantageusement, on choisit les nanoparticules de sorte qu'elles ont une relaxivité ($r_1$) par particule comprise allant de 50 à 5000 $mM^{-1}.s^{-1}$ et/ou un rapport massique en ions métalliques d'au moins 5%, par exemple allant de 5 à 50%. De préférence, les nanoparticules ont une relaxivité $r_1$ par particule comprise allant de 50 à 5000$mM^{-1}.s^{-1}$ et/ou un rapport massique en $Gd^{3+}$ d'au moins 5%, par exemple allant de 5 à 50%, avantageusement allant de 10% et 15%. Le rapport massique en $Gd^{3+}$ correspond à la masse en $Gd^{3+}$ par rapport à la masse de la nanoparticule.

La matrice POS a un diamètre qui peut alors aller de 0,5 à 8 nm, avantageusement de 1 à 5 nm, et représenter de 25 à 75% du volume total des nanoparticules. Elle permet notamment le greffage du ou des agents chélatants.

Dans un mode de réalisation particulier, les nanoparticules comprennent en outre une ou plusieurs agents fonctionnalisants, greffés à la matrice POS ou à l'agent chélatant.

Le ou les agents fonctionnalisants peuvent être choisis parmi :

- Une ou plusieurs molécules ciblantes pour le ciblage des nanoparticules. Ces molécules ciblantes permettent un ciblage des nanoparticules vers des tissus spécifiques et/ou des cellules spécifiques. Elles peuvent par exemple cibler les tissus rénaux, par exemple les tissus du cortex rénal, la médulla rénale interne ou la médulla rénale externe. De cette façon, on concentre les nanoparticules dans la zone d'intérêt sans avoir à augmenter de manière très importante les quantités administrées ;
- Un ou plusieurs agents fluorescents ;
- Un ou plusieurs polymères hydrophiles, de préférence du polyéthylène glycol (PEG). Les composés hydrophiles de type PEG permettent d'adapter la biodistribution des nanoparticules au sein de l'organisme. Le ou les polymères hydrophiles permettent une plus grande furtivité et/ou neutralité des nanoparticules. Ce type de revêtement permet notamment de réduire les éventuels phénomènes de néphrotoxicité associés aux nanoparticules;
- Une combinaison de ces agents.

Le ou les agents fonctionnalisant sont greffés à la matrice POS ou à l'agent chélatant. On pourra utiliser un couplage classique avec des groupes réactifs présents au niveau des nanoparticules et de l'agent fonctionnalisant, éventuellement précédé d'une étape d'activation. Les réactions de couplage sont connues de l'homme du métier et seront choisies en fonction de la structure des nanoparticules et des groupements fonctionnels du ou des agents fonctionnalisant. Voir par exemple, « Bioconjugate Techniques », G.T Hermanson, Académie Press, 1996, dans « Fluorescent and Luminescent Probes for Biological Activity », Second Edition, W.T. Mason, ed. Académie Press, 1999. De préférence, ces agents fonctionnalisants sont greffés aux agents chélatants des nanoparticules.

Selon une variante de l'invention, les nanoparticules peuvent être fonctionnalisées à leur surface avec des composés hydrophiles choisis dans le groupe des polyols, les sucres, les dextranes et leurs mélanges. Les glycols, avantageusement le PEG, étant particulièrement préférés. Suivant une alternative, ces composés hydrophiles peuvent être choisis parmi ceux qui ont des masses molaires inférieures à 2000 g/mol, de préférence inférieures à 800 g/mol.

On choisira le ou les agents fonctionnalisants en fonction de l'application envisagée.

L'un des intérêts majeurs des nanoparticules selon l'invention, est qu'elles peuvent être utilisées comme agent d'imagerie dans des systèmes d'imagerie, comme par exemple l'IRM, la scintigraphie TEMP, la scintigraphie TEP, l'imagerie par fluorescence, l'imagerie aux rayons X.

De préférence, les nanoparticules selon l'invention, ont une relaxivité ($r_1$) par ion $M^{n+}$ supérieure à 5 $mM^{-1}$ d'ion $M^{n+}.s^{-1}$, préférentiellement 10 $mM^{-1}$ d'ion $M^{n+}.s^{-1}$ pour une fréquence de 20 MHz. Par exemple, elles ont une relaxivité ($r_1$) par nanoparticule compris allant de 50 à 5000 $mM^{-1}.s^{-1}$. Par exemple, ces nanoparticules ont une relaxivité ($r_1$) par ion $M^{n+}$ à 60 MHz qui est supérieure ou égale à la relaxivité ($r_1$) par ion $M^{n+}$ à 20 MHz. La relaxivité ($r_1$) considérée ici est une relaxivité par ion $M^{n+}$ (par exemple $Gd^{3+}$). $r_1$ est extrait de la formule suivante : $1/T_1 = [1/T_1]_{eau} + r_1[M^{n+}]$.

T1 correspond au temps de relaxation longitudinal exprimé en seconde (s). Il s'agit du temps au bout duquel l'aimantation basculée dans le plan lors d'une excitation de résonance magnétique nucléaire (RMN) revient à son état d'équilibre, par interactions dipolaires spin réseau avec les noyaux voisins (i.e. spins de l'eau). Par définition $T_1$, est l'intervalle de temps (en milliseconde ou seconde) correspondant à la récupération de 63% de la magnétisation longitudinale initiale. $T_1$ dépend des propriétés des noyaux d'hydrogène contenus dans les différents tissus. $T_1$ varie selon les tissus.

$[1/T_1]_{eau}$ correspond au temps de relaxation longitudinal de l'eau sans agents d'imagerie. Cela correspond à l'inverse du temps de relaxation longitudinal en absence d'agent d'imagerie, exprimé en $seconde^{-1}$ ($s^{-1}$).

$r_1$ correspond au taux de relaxation, exprimé en $s^{-1}$. Cette valeur correspond au retour du spin du proton à son état antérieur, i.e. après l'excitation par l'impulsion RF.

$[M^{n+}]$ correspond à la concentration en ion métallique, exprimée en $mM^{-1}$.

$r_1[M^{n+}]$ correspond à la relaxivité longitudinale de l'ion métallique exprimée en $mM^{-1}.s^{-1}$ ou en $M^{-1}.s^{-1}$ Cette valeur reflète l'efficacité d'un agent d'imagerie IRM de type $T_1$ normalisé avec la concentration en ion métallique. On définit aussi la relaxivité $r_2$ en considérant dans la formule le temps de relaxation T2.

A titre indicatif, il est également possible de calculer le rapport $r_2/r_1$ qui doit être proche de 1 pour assurer la qualité d'agent de type $T_1$ en IRM.

Dans une variante, les nanoparticules ont un rapport massique en ion métallique IRM $T_1$ de type lanthanide, de préférence en $Gd^{3+}$, supérieur ou égal à 5%, par exemple allant de 5% à 50%. Le rapport massique en ions métalliques correspond à la masse en ions métalliques par rapport à la masse de la nanoparticule.

Plus de détails concernant ces nanoparticules ultrafines, leurs procédés de synthèse et leurs applications figurent dans la demande de brevet WO2011/135101, qui est incorporée par référence, et dans la publication Mignot et al (A Top-Down Synthesis Route to Ultrasmall Multifunctionnal Gd-Based Silica Nanoparticles for Theranostics Applications, Chem. Eur. J., 2013, 19, 6122-6136), qui est également incorporée par référence.

Selon l'invention, les nanoparticules selon l'invention présentent un diamètre moyen hydrodynamique moyen inférieur à 10 nm, avantageusement inférieur à 5 nm, par exemple allant de 1 à 5 nm. Ce très faible diamètre permet une excellente répartition des nanoparticules dans les reins et donc la détection fiable d'une dysfonction rénale.

La distribution de taille des nanoparticules est par exemple mesurée à l'aide d'un granulomètre commercial, tel qu'un granulomètre Malvern Zêta sizer Nano-S basé sur la PCS (Photon Correlation Spectroscopy). Cette distribution est caractérisée par un diamètre hydrodynamique moyen.

Au sens de l'invention, par « diamètre hydrodynamique moyen» on entend la moyenne harmonique des diamètres des particules. Une méthode de mesure de ce paramètre est également décrite dans la norme ISO 13321: 1996.

L'invention vise également l'utilisation des nanoparticules selon l'invention, comme agent d'imagerie dans le diagnostic *in vivo* d'une dysfonction rénale, par exemple une fibrose rénale et/ou une insuffisance rénale, chez l'homme ou l'animal. La dysfonction rénale peut également être une diminution de la fonctionnalité rénale, en particulier une diminution de la fonction tubulaire et/ou une diminution de la filtration glomérulaire. La dysfonction rénale peut évoluer en fibrose rénale. Dans les cas les plus graves, notamment dans les cas les plus graves de fibrose rénale, la dysfonction rénale évolue en insuffisance rénale, notamment en insuffisance rénale chronique.

L'invention vise également l'utilisation des nanoparticules selon l'invention, pour la préparation d'une composition destinée au diagnostic d'une dysfonction rénale, par exemple une fibrose rénale, par exemple une insuffisance rénale, chez l'homme ou l'animal. La fibrose rénale peut être liée à une atteinte des tubules rénaux et/ou des glomérules.

De préférence, la composition est une composition pharmaceutiquement acceptable, adaptée pour une administration par voie intraveineuse ou par voies aériennes, de préférence par voie intraveineuse.

Ainsi, l'invention concerne également des compositions comprenant des nanoparticules selon l'invention. Les compositions comprennent des nanoparticules selon l'invention et un ou plusieurs excipients pharmaceutiquement acceptables.

L'invention vise également une méthode pour diagnostiquer une dysfonction rénale *in vivo* chez l'homme ou l'animal auquel ont été administrées des nanoparticules telles que définies ci-dessus. Dans un mode de réalisation particulier, la méthode comprend les étapes de :

(i) capture des images par une technique d'imagerie appropriée afin de visualiser au niveau du rein des nanoparticules selon l'invention ;
(ii) détermination du rehaussement ;
(iii) comparaison du rehaussement déterminé à l'étape (ii) à un rehaussement de référence.

Dans un mode de réalisation particulier, la méthode de diagnostic est mise en oeuvre chez un patient auquel ont déjà été administrées lesdites nanoparticules, c'est-à-dire que ladite méthode de diagnostic ne comprend pas d'étape d'administration desdites nanoparticules.

La comparaison du rehaussement déterminé à l'étape (ii) à un rehaussement de référence permet de diagnostiquer la dysfonction rénale, par exemple de diagnostiquer la fibrose rénale, par exemple de diagnostiquer l'insuffisance rénale.

Dans un mode de réalisation particulier, la méthode comprend une capture des images par IRM (i.e. étape i) et comprend également une étape (iv) mesure de la concentration en agent d'imagerie IRM par des cartographies quantitatives $T_1$. La cartographie quantitative correspond à une séquence d'acquisition spécifique déterminée par l'homme de métier, caractérisée par une pondération $T_1$ adaptée à l'agent d'imagerie, une rapidité d'enregistrement compatible avec la résolution cinétique de l'évolution du signal et la qualité de résolution spatiale de l'image, une robustesse au mouvement. La cartographie quantitative peut être transposée à d'autres techniques d'imagerie appropriées.

Au sens de l'invention, par « rehaussement » ou « rehaussement d'un signal d'imagerie » on entend la variation du signal d'imagerie, par exemple du signal IRM, lié à l'arrivée de l'agent d'imagerie dans le tissu d'intérêt. Selon l'invention, le rehaussement correspond à la variation du signal d'imagerie lié à l'arrivée des nanoparticules au niveau du rein, avantageusement au niveau du cortex rénal, de la médulla rénale interne et/ou de la médulla rénale externe, c'est-à-dire à la différence entre le niveau du signal avant injection des nanoparticules et le niveau du signal après injection des nanoparticules. Le rehaussement est un paramètre bien connu de l'homme du métier. En général, les dispositifs d'imagerie communément utilisés, par exemple par les dispositifs d'IRM, proposent des outils de traitement d'images permettant de mesurer le rehaussement du tissu ou de l'organe étudié.

Le rehaussement obtenu au niveau du rein avec les produits d'imagerie classiquement utilisés, e.g. le DOTAREM en IRM, ne permet pas de distinguer un rein sain d'un rein atteint d'une dysfonction rénale. Les inventeurs ont mis en évidence que le rehaussement des nanoparticules selon l'invention au niveau du rein, avantageusement au niveau du cortex rénal, de la médulla rénale interne et/ou de la médulla rénale externe, permet de distinguer un rein sain d'un rein atteint de dysfonction rénale. Ainsi, les nanoparticules selon l'invention permettent de diagnostiquer une dysfonction rénale.

Le rehaussement se calcule en faisant le rapport suivant :

$$r(t) = [S(t) - S0]/S0$$

r(t) correspond à un taux de rehaussement à un temps t ; S0 correspond au niveau de signal avant l'arrivée de l'agent d'imagerie dans le tissu ou l'organe d'intérêt, aussi appelé niveau de signal précontraste ; S(t) correspond au niveau de signal à un temps t, qui correspond à un niveau de signal lorsque l'agent d'imagerie se trouve au niveau du tissu d'intérêt.

Selon l'invention, la détermination du rehaussement se fait par (i) la détermination du pic de rehaussement et/ou (ii) l'obtention d'une courbe de rehaussement.

Le « pic de rehaussement » correspond au rehaussement maximum obtenu après injection de l'agent d'imagerie, c'est-à-dire lorsque le signal d'imagerie est maximal. Généralement, le temps après injection au bout duquel est atteint le pic de rehaussement est commun à tous les individus d'une même espèce. Par exemple chez l'homme, le temps après injection pour lequel est atteint le pic de rehaussement est d'environ 10 minutes, par exemple allant de 5 à 15 minutes, par exemple allant de 8 à 12 minutes. Le temps après injection au bout duquel est atteint le pic de rehaussement pour une espèce donnée peut facilement être déterminé par l'homme du métier, notamment en fonction de la région d'intérêt observée, par exemple le cortex rénal, les tubules rénaux la médulla rénale interne et/ou la médulla rénale externe.

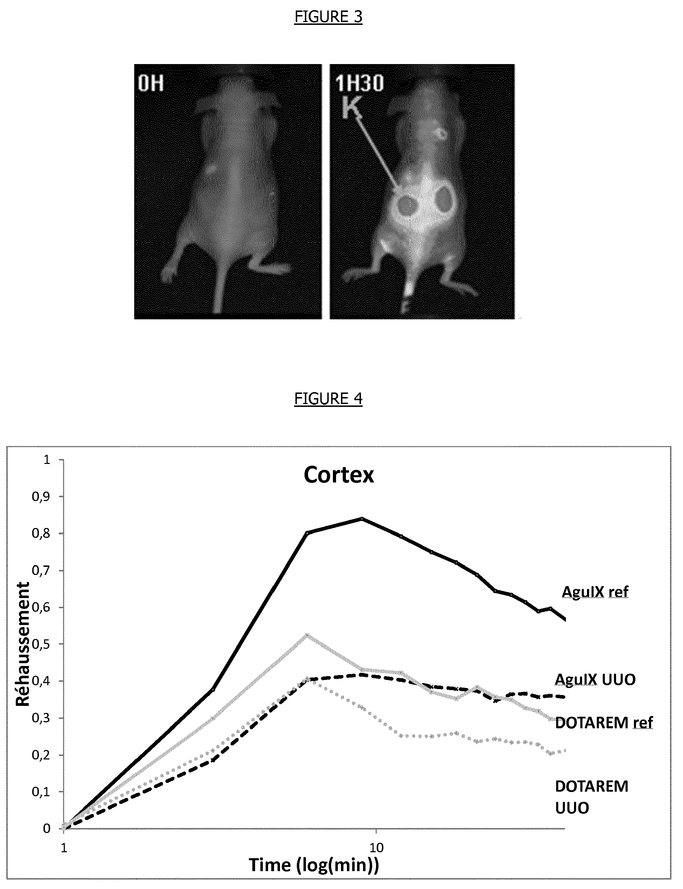

La « courbe de rehaussement » correspond à l'évolution du rehaussement au cours du temps. A titre d'exemple, les figures 4, 5 et 6 correspondent à des courbes de rehaussement obtenues au niveau du cortex rénal de souris. La courbe de rehaussement permet de visualiser l'arrivée et l'élimination au cours du temps de l'agent d'imagerie au niveau du tissu d'intérêt.

Dans un mode de réalisation particulier, le diagnostic de la dysfonction rénale se fait en comparant le rehaussement à un rehaussement de référence. Par exemple, le rehaussement de référence correspond au rehaussement d'un rein sain, généralement similaire pour tous les individus d'une même espèce.

Selon un mode de réalisation particulier, la détermination du pic de rehaussement selon l'invention permet de déterminer si le rein est sain ou atteint d'une dysfonction rénale. Ainsi, une baisse du pic de rehaussement supérieure à 5%, par exemple supérieure à 6%, 7%, 8%, 9% ou supérieure à 10%, par exemple supérieure à 15%, 20%, 25%, 30%, 35%, 40%, 45% ou supérieure à 50% par rapport au pic de rehaussement de référence est le reflet d'une dysfonction rénale.

Selon un autre mode de réalisation, la courbe de rehaussement peut également donner des informations exploitables au sens de l'invention, e.g. (i) temps de diffusion et (ii) temps d'élimination de l'agent d'imagerie. Ainsi, la courbe de rehaussement peut permettre également de diagnostiquer une dysfonction rénale. En particulier, la courbe de rehaussement est comparée à une courbe de rehaussement de référence, ladite courbe de rehaussement de référence pouvant être déterminée aisément, par exemple en mesurant l'évolution du rehaussement au cours du temps chez un individu sain.

Une application particulière de la présente invention porte sur le suivi de l'efficacité thérapeutique d'un traitement chez l'homme ou l'animal, par exemple d'un traitement d'une dysfonction rénale, en particulier d'un traitement d'une fibrose rénale, en particulier d'un traitement d'une insuffisance rénale. Ainsi, l'invention vise une méthode de suivi de l'efficacité thérapeutique d'un traitement d'une dysfonction rénale chez l'homme ou l'animal auquel ont été administrées des nanoparticules selon l'invention, en particulier d'une fibrose rénale, en particulier une insuffisance rénale, ladite méthode comprenant les étapes suivantes :

(i) on capture des images par une technique d'imagerie appropriée afin de visualiser au niveau du rein des nanoparticules selon l'invention,
(ii) on détermine le rehaussement ;
(iii) on répète les étapes (i) et (ii) au cours du traitement du sujet, autant que nécessaire ;
(iv) on déduit l'efficacité thérapeutique du traitement en comparant l'évolution du rehaussement au cours du traitement.

L'invention vise également une méthode de suivi de l'efficacité thérapeutique d'un traitement d'une dysfonction rénale chez l'homme ou l'animal qui comprend les étapes suivantes :

(i) on administre au patient, des nanoparticules selon l'invention, à titre d'agent d'imagerie,
(ii) on capture des images par une technique d'imagerie appropriée afin de visualiser au niveau du rein lesdites nanoparticules
(iii) on détermine le rehaussement ;
(iv) on répète les étapes (i) et (iii) au cours du traitement du sujet, autant que nécessaire ;
(v) on déduit l'efficacité thérapeutique du traitement en comparant l'évolution du rehaussement au cours du traitement.

Ainsi, on peut suivre l'évolution de la dysfonction rénale au cours du temps, avant, pendant, et après le traitement du patient.

Dans un mode de réalisation particulier, le rehaussement est déterminé avant, pendant et/ou après le traitement. Cela permet de comparer l'évolution du rehaussement au cours du traitement de la dysfonction rénale. Selon un mode de réalisation préféré, on compare l'évolution du rehaussement par rapport au rehaussement déterminé avant le traitement, considéré alors comme le rehaussement de référence. Ainsi, le suivi du rehaussement permet de suivre l'efficacité thérapeutique d'un traitement d'une dysfonction rénale chez l'homme ou l'animal.

Selon un mode de réalisation particulier, la détermination du pic de rehaussement avant, pendant et/ou après le traitement permet de déterminer l'efficacité thérapeutique du traitement d'une dysfonction rénale. Ainsi, une augmentation du pic de rehaussement supérieure à 5%, par exemple supérieure à 6%, 7%, 8%, 9% ou supérieure à 10%, par exemple supérieure à 15%, 20%, 25%, 30%, 35%, 40%, 45% ou supérieure à 50% par rapport au pic de rehaussement de référence reflète l'efficacité thérapeutique du traitement de la dysfonction rénale.

La courbe de rehaussement peut également donner des informations exploitable au sens de l'invention, e.g. (i) temps de diffusion et (ii) temps d'élimination de l'agent d'imagerie. Ainsi, la courbe de rehaussement peut également permettre de déduire l'efficacité thérapeutique du traitement d'une dysfonction rénale. En particulier, la courbe de rehaussement est comparée à une courbe de rehaussement de référence, par exemple à la courbe de rehaussement déterminée avant traitement.

Dans un mode de réalisation préféré, les nanoparticules selon l'invention sont visualisées au niveau du cortex rénal, de la médulla rénale interne et/ou de la médulla rénale externe. Les inventeurs ont en effet montré que la visualisation des nanoparticules selon l'invention au niveau du cortex rénal permet une détermination optimale du rehaussement, et ainsi permettre un diagnostic précis de la dysfonction rénale ou un suivi précis de l'efficacité thérapeutique du traitement d'une dysfonction rénale.

L'invention porte également sur une méthode pour traiter une dysfonction rénale chez l'homme ou l'animal, comprenant les étapes suivantes:

(i) administrer des nanoparticules selon l'invention à un homme ou un animal par voie intraveineuse à une dose comprise dans la gamme allant de 5 $\mu$mol/kg à 50 $\mu$mol/kg ;
(ii) capturer des images par une technique d'imagerie appropriée afin de visualiser au niveau du rein lesdites nanoparticules, de préférence de 0 à 24 heures après l'étape d'administration ;
(iii) mesurer le rehaussement ;
(iv) lorsque la mesure de l'étape (iii) permet de diagnostiquer une dysfonction rénale, instaurer un traitement approprié chez l'homme ou l'animal.

Dans un mode de réalisation particulier, le traitement approprié correspond à l'administration d'une quantité suffisante d'un médicament permettant de traiter la dysfonction rénale.

En particulier, le traitement approprié peut être choisi parmi les traitements suivants ou une combinaison de ceux-ci :

- Les traitements standards des pathologies induisant une dysfonction rénale, par exemple les traitements du diabète, de l'hypertension ou des maladies auto-immunes ;
- Les inhibiteurs du système rénine angiotensine (RSA), par exemple les antihypertenseurs antagonistes des récepteurs de l'angiotensine II ou les inhibiteurs de l'enzyme de conversion responsable de la production d'angiotensine II;
- Les inhibiteurs de cytokines profibrosantes, par exemple les inhibiteurs de TGF-β, CTGF, PDGF, comme la Pirfénidone (développé par Roche-InterMune), le FG-3019 (développé par FibroGen/BMS), le Fresolimumab (développé par Sanofi), le THR-184 (développé par Thrasos therapeutics) ;
- D'autres molécules avec différents modes d'action, par exemple l'inhibiteur de NOX KGT1377831 (développé par Genkyotex), l'inhibiteur de CCR2 CCX-140 (développé par Chemocentryx), l'inhibiteur de ALK5 SB525334 (développé par GSK), l'anti $\alpha 5\beta 6$ integrin STX-100 / Biogen Idec, ou l'inhibiteur de LPA1 BMS-986020 (développé par BMS) ;
- La dialyse ou la transplantation rénale.

Dans un mode de réalisation particulier, l'étape (ii) de capture des images par une technique d'imagerie appropriée est réalisée toutes les 3 minutes de 0 à 40 min puis toutes les 4h jusqu'à 24h.

Dans un mode de réalisation particulier de l'invention, l'étape de mesure du rehaussement est pratiquée sur un seul rein ou sur les deux reins. En particulier, le rehaussement peut être mesuré pour chacun des reins.

Lorsque l'ion métallique est le gadolinium ($Gd^{3+}$), les nanoparticules selon l'invention sont administrées par voie intraveineuse à une dose inférieure à 0,1 mM de Gd/kg. Dans un mode de réalisation particulier, les nanoparticules sont administrées à une dose comprise de 0,001 à 0,1 mM de Gd/kg, avantageusement de 0,01 à 0,05 mM de Gd/kg, avantageusement de 0,01 à 0,02 mM de Gd/kg, avantageusement à une dose de 0,017 mM de Gd/kg.

Les nanoparticules selon l'invention peuvent être administrées par voie systémique, avantageusement par voie intraveineuse ou par voie aérienne. La demande de brevet WO2013/153197 décrit l'administration de nanoparticules ultrafines par les voies aérienne, lesdites nanoparticules étant capables de diffuser dans la circulation sanguine avant d'être éliminées par le rein.

Légendes des figures

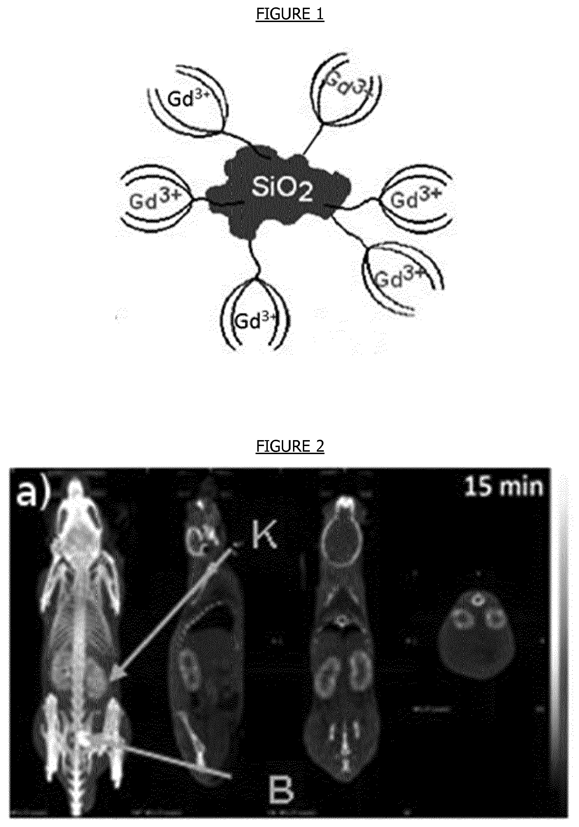

*Figure 1:* Représentation schématique d'une nanoparticule selon l'invention. Cette nanoparticule comprend une matrice polyorganosiloxane sur laquelle sont greffés 6 agents chélatants de type DOTAGA, 6 agents chélatants ayant chélaté des ions Gadolinium $Gd^{3+}$.

*Figure 2 :* Image de coupes longitudinales en SPECT/CT d'une souris c57Bl/6J male 15 minutes après injection intraveineuse des nanoparticules obtenues suivant l'exemple 1 et couplées à de l'indium 111. K correspond au rein ; B correspond à la vessie. Ces images montrent que les nanoparticules sont éliminées par les reins et ne s'accumulent pas dans le foie.

*Figure 3* : imagerie de fluorescence par réflectance de souris femelles "swiss nude" avant (gauche) et après (droite) l'administration intraveineuse des nanoparticules obtenues suivant l'exemple 1 et couplées à un fluorophore de type Cy5.5. K correspond au rein. Ces images montrent que les nanoparticules sont éliminées par les reins et ne s'accumulent pas dans le foie.

*Figure 4:* comparaison du rehaussement obtenu par IRM après administration intraveineuse (i) de nanoparticules obtenues suivant l'exemple 1 ou (ii) de nanoparticules DOTAREM chez une souris saine ou une souris UUO. Les résultats montrent que les nanoparticules DOTAREM ne peuvent pas discriminer entre rein sain et rein lésé alors qu'une différence significative est obtenue avec les nanoparticules obtenues suivant l'exemple 1.

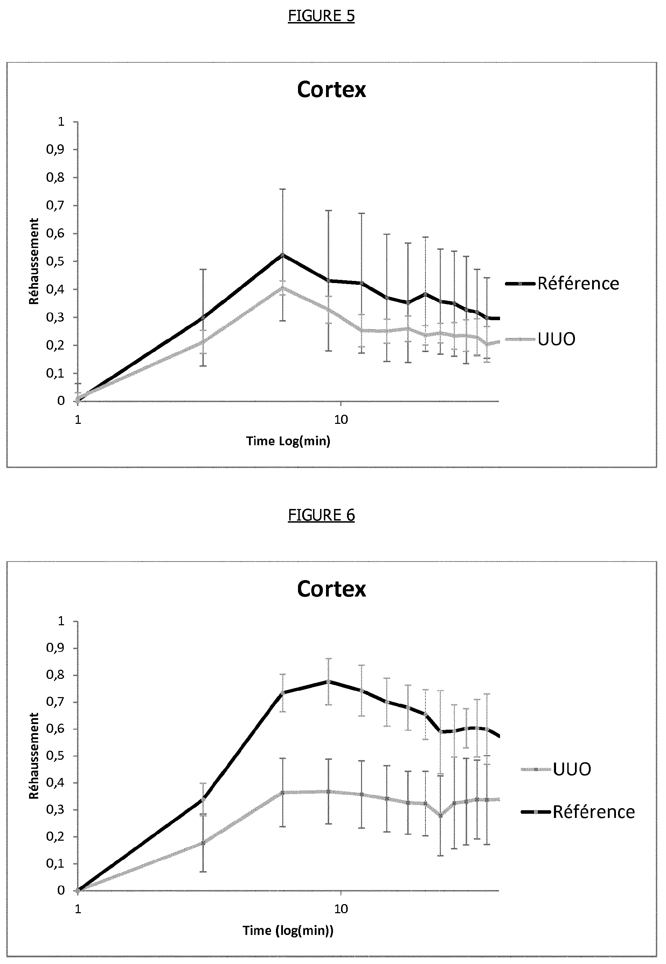

*Figure 5:* cinétique de la mesure du rehaussement au niveau du cortex rénal par IRM après administration par voie intraveineuse de nanoparticules DOTAREM (10 mM). Les courbes obtenues montrent que le profil de rehaussement est similaire entre rein UUO (rein lésé) et rein référence (rein sain). Les résultats montrent que les nanoparticules DOTAREM ne peuvent pas discriminer entre rein sain et rein lésé.

*Figure 6:* cinétique de la mesure du rehaussement au niveau du cortex rénal par IRM après administration par voie intraveineuse de nanoparticules obtenues suivant l'exemple 1 (5 mM). Les courbes obtenues montrent que le profil de rehaussement est significativement différent entre rein UUO (rein lésé) et rein référence (rein sain). Les résultats montrent que les nanoparticules obtenues suivant l'exemple 1 peuvent discriminer entre rein sain et rein lésé.

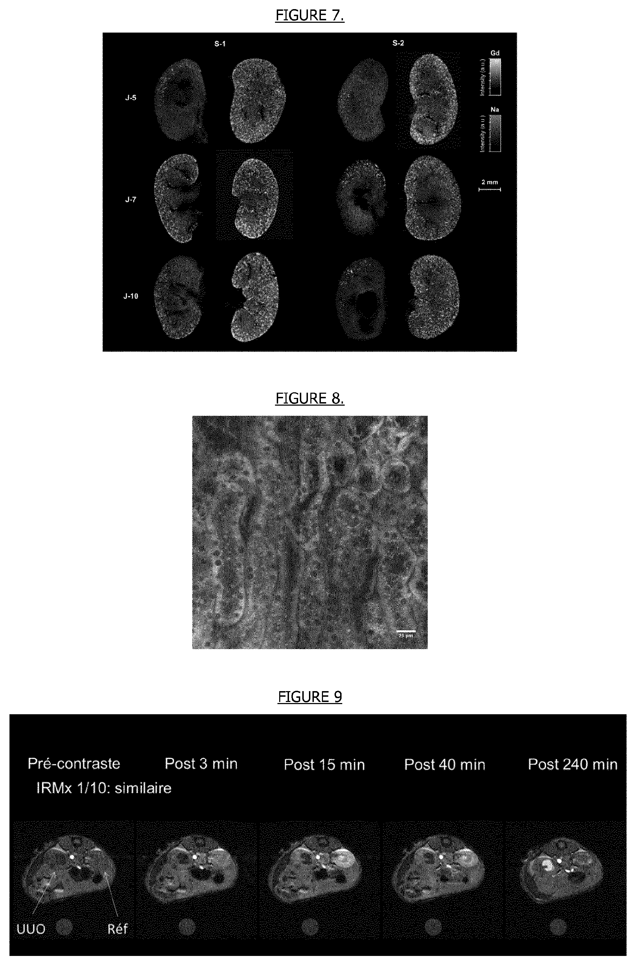

*Figure 7*: Images obtenues pour visualisation de nanoparticules obtenues suivant l'exemple 1 (nanoparticules AGuIX) dans les reins par LIBS (Laser-Induced Breakdown Spectroscopy). Le LIBS permet de visualiser les atomes de gadolinium provenant des AGuIX, représentés en vert, et ceux de sodium (en rouge) répartis de manière homogène dans le tissu. Les résultats montrent que le rein déficient ne retient pas ou retient peu les nanoparticules, ce qui est en accord avec les résultats obtenus par IRM *in vivo*.

*Figure 8:* Images histologiques obtenues en Microscopie Photonique confocale permettant de visualiser des selon l'invention greffées avec un chromophore Cy5.5 (i.e. nanoparticules AGuIX greffées avec un chromophore Cy5.5, en rouge) dans les cellules tubulaires rénales. Les nanoparticules sont accumulées dans les tubules rénaux, étape transitoire préalable à leur élimination.

*Figure 9:* Images obtenues par IRM dynamique des reins d'une souris (1 rein UUO et 1 rein normal) avant et après injection de nanoparticules selon l'invention. Les résultats montrent que le rein déficient ne retient pas ou retient peu les nanoparticules selon l'invention. Ainsi, les images montrent que le rehaussement au niveau du cortex rénal du rein de référence est beaucoup plus important qu'au niveau du cortex rénal du rein UUO.

## **Exemples**

Exemple 1 : Préparation de nanoparticules de type DOTAGA

Synthèse des nanoparticules

Une solution a été préparée en dissolvant 167,3 g de [GdCl$_3$, 6 H$_2$O] dans 3 L de diethylène glycol (DEG) à température ambiante. Le mélange a ensuite été mis sous agitation pendant 3 heures à 140°C. On a ensuite ajouté 44,5 mL de soude à 10 M puis chauffé 5 heures sous agitation à 180°C pour obtenir la solution A. Les coeurs d'oxyde de gadolinium obtenus dans la solution A présentent un diamètre hydrodynamique de 1,7 ±0.5 nm.

La couche de polysiloxane a été obtenue par procédé sol-gel (i.e. par réactions d'hydrolyse condensation en conditions basiques obtenues par ajout de précurseurs organosilane). Dans un premier temps, une première solution contenant 1,6 L de DEG, 51,4 mL de tetraethoxysilane (TEOS) et 80,6 mL d'aminopropyltriéthoxysilane (APTES) a été ajoutée lentement (i.e. en 96 heures) à la solution A à une température de 40°C. Une heure après l'addition de la première solution, une seconde solution contenant 190 mL de DEG, 43,1 mL d'eau et 6,9 mL de triethylamine (TEA) a été ajoutée sous agitation pendant 96 heures à 40°C. A la fin de la réaction, le mélange a été laissé sous agitation 72 heures à 40°C puis 12 heures à température ambiante pour obtenir la solution B. Les coeurs d'oxyde de gadolinium recouverts de polysiloxane de la solution B présentent un diamètre hydrodynamique de 2,6 ± 1,0 nm.

163,7 g de DOTAGA anhydre ont été ensuite ajoutés à la solution B. La solution résultante a été laissée sous agitation 72 heures à température ambiante pour obtenir la solution C qui contient des nanoparticules comprenant un coeur d'oxyde de gadolinium recouvert de polysiloxane sur lesquelles sont greffés des groupements DOTAGA.

*Purification*

17,5 L d'acétone ont été ajoutés à la solution C pour précipiter les nanoparticules qui ont été récupérées par filtration sous vide. Les nanoparticules ont ensuite été redispersées dans de l'eau et placées à pH 2 pendant 1 heure. L'acétone restante a été évaporée. Les nanoparticules ont ensuite été purifiées par filtration tangentielle sur une membrane de 5 kDa avant l'ajout de soude pour obtenir un pH de 7,4. Le passage dans l'eau et la purification dans l'eau a entraîné la dissolution du coeur d'oxyde de gadolinium. Cette dissolution du coeur d'oxyde de gadolinium est favorisée par la présence des groupements chélatants greffés à la matrice polysiloxane de la nanoparticule qui complexent le gadolinium libéré par la dissolution du coeur. Une fois le coeur dissous, la coquille de polysiloxane s'est fragmentée en nanoparticules ultrafines constituées d'une matrice de polysiloxane sur laquelle sont greffés des DOTAGA qui ont chélaté du gadolinium. La solution ainsi obtenue a ensuite été filtrée deux fois par un filtre de 1,2 $\mu$m puis par un filtre de 0,2 $\mu$m afin d'éliminer les impuretés les plus massives. Les nanoparticules ainsi obtenues présentent un diamètre hydrodynamique de 2,2 ± 1.0 nm, une relaxivité longitudinale de 11,5 mmol$^{-1}$.L.s$^{-1}$ à 60 MHz et à 37°C. Environ 50 grammes de nanoparticules ont été obtenues à la fin de la synthèse.

Exemple 2 : Elimination des nanoparticules par le rein

Protocole d'imagerie élémentaire LIBS (Laser induced breakdown spectroscopy)

Les nanoparticules obtenues suivant l'exemple 1 ont été administrées par voie intraveineuse à des souris anesthésiées (8 mg/souris). Celles-ci ont ensuite été sacrifiées à 15 min et à 1h30 afin de permettre le prélèvement des reins.

Les reins ont été perfusés et fixés dans un tampon glutarahdéhyde 2% préparé dans une solution de cacodylate de sodium à 0,1M (pH 7,4) pendant une nuit, à 4°C.

Puis les reins ont été rincés dans une solution de cacodylate de sodium à 0,2M (pH 7,4). Après fixation, les reins ont été déshydratés par différents bains d'éthanol de concentration croissante de 30% à 100% d'éthanol comme suit : 30%, 50%, 70%, 80%, 95%, 100%, 100%, 100%. Chaque bain a duré 30min.

Puis les reins ont été plongés dans une solution d'oxyde de propylène / éthanol 100% (1:1) pendant 30 min, puis dans 2 bains d'oxyde de propylène (30min chacun).

Lors de l'imprégnation, les reins ont été plongés dans différents bains d'oxyde de propylène et de résine EPON de 4 heures à 1 nuit selon les ratios (2:1), (1:1), (1:2), (0:1), (0:1).

Puis les reins ont été plongés dans un mélange d'EPON fraichement préparé et contenant le durcisseur et placés à l'étude à 56°C pendant 72h.

L'échantillon peut alors être préparé pour l'analyse LIBS par coupe et polissage de la surface du rein.

Les résultats sont présentés sur les figures 7 et 8.

Conclusion : le rein déficient ne retient que très peu les nanoparticules à l'inverse du rein fonctionnel qui retient les nanoparticules.

Exemple 3 : Mesure du rehaussement au niveau du rein par IRM : comparaison entre des nanoparticules selon l'invention (nanoparticules AGuIX) et les nanoparticules de l'art antérieur (DOTAREM).

Les images ont été acquises avec un spectromètre imageur 7T 300 MHz, équipé d'une bobine radiofréquence 1H linéaire dédiée pour la souris (Bruker, Karlsruhe Allemagne). Les études IRM dynamique de capture et relargage des nanoparticules dans les tissus ont été réalisées in vivo et ont permis de déterminer l'évolution du rehaussement au niveau du rein.

Toutes les manipulations des animaux ont été réalisée en conformité avec les directives institutionnelles de protocole animal en place à l'Université Paris Descartes, saisine CEEA34.JS.142.1 et approuvé par le Comité de recherche animale de l'Institut, ainsi que les protocoles éthiques de la société concernant les inductions des souris. Les relaxivités en solutions ont été préalablement mesurées à 7T pour préparer la concentration finale d'injection.

Des souris femelle de type BalbC/JRJ de 8 semaines (n=6) ont été traitées UUO au niveau d'un seul rein, i.e. chaque souris comporte un rein lésé (rein UUO) et un rein sain (rein référence). Les souris ont été anesthésiées par inhalation d'isoflurane (1,5% d'air / $O_2$ 0,5 / 0,25l / min) et placées dans un berceau de contention dédié. Les paramètres respiratoires physiologiques ont été mesurés tout au long des études IRM par un système de capteurs respiratoires amagnétiques (société SAM, US) et le maintien de la température corporelle a été assuré par un chauffage au coeur du berceau.

Deux solutions ont été préparées :

- Solution selon l'invention : 100 μl d'agent d'imagerie solubilisé dans une solution saline à 0,9%, avec une concentration de 5 mM en agent d'imagerie AguIX (i.e. les nanoparticules obtenues suivant l'exemple 1).

- Solution selon l'art antérieur : 100 μl d'agent d'imagerie solubilisé dans une solution saline à 0,9%, avec une concentration de 10mM en agent d'imagerie DOTAREM.

Les solutions ont été administrées à des souris par voie intraveineuse via la veine caudale grâce à un cathéter (spécifiquement élaboré pour une utilisation non magnétique; calibré avec volume mort connu), alors que la souris se trouvait dans le scanner.

Pour les études de reproductibilité, 100μL d'une la solution de nanoparticules AGuIX à 5mM a été administrée aux 6 souris (le rein sain étant le rein de référence ou réf., et le rein lésé étant le rein UUO)

Le protocole d'acquisition original a été développé avec le logiciel Paravision 5.1.

Une séquence améliorée de contraste dynamique DCE a été enregistrée à l'aide de d'une séquence spécifique supprimant les artefacts libres de mouvement en post traitement avec un TR de 100 ms et un TE de 4ms et un angle de basculement de 80 ° pour assurer une pondération IRM $T_1$. Un champ de vue (FOV) de 3 cm x 3 cm et une matrice finale de 256 par 256 points. 4 tranches d'une épaisseur de 1 mm, ont été choisies ce qui donne une résolution spatiale planaire de 117μm x 117μm. Le temps total d'une acquisition par image a été de l'ordre de 3 min 14sec. Enfin, une version allongée de la séquence basée sur la répétition de la séquence précédente a été utilisée pour le suivi dynamique pour obtenir la même résolution temporelle en un temps de cycle de 40 min à 4h.

Après l'acquisition des images, un traitement des images a été effectué en délimitant des régions rénales d'intérêt dans les zones corticales, médullaires ou autres. Les intensités obtenues au niveau de la zone corticale ont été reportées sur un fichier Excel et le rehaussement $(S_0 - S) / S_0$ a été reporté sur un graphique en fonction du temps (min) auquel l'image a été acquise. Les résultats sont présentés aux figures 4 à 6.

Conclusion : Les nanoparticules selon l'invention permettent de différencier, de manière significative, la fonctionnalité rénale entre un rein sain et un rein obstrué. Le DOTAREM ne permet pas de différencier, de manière significative, la fonctionnalité rénale d'un rein sain et d'un rein obstrué.

**Revendications**

1. Nanoparticules destinées à être utilisées comme agent d'imagerie dans une méthode de diagnostic *in vivo* d'une dysfonction rénale chez l'homme ou l'animal, lesdites nanoparticules comprenant :

 (i) une matrice de polyorganosiloxane (POS) ;
 (ii) un ou plusieurs agents chélatants, de préférence de 6 à 20 agents chélatants, greffés à la matrice POS par liaison covalente -Si-C- ;

(iii) un ou plusieurs ions métalliques chélatés par un ou plusieurs des agents chélatants susmentionnés.

2. Nanoparticules destinées à une utilisation selon la revendication 1, **caractérisées en ce que** lesdites nanoparticules présentent un diamètre hydrodynamique moyen inférieur à 10 nm, de préférence allant de 1 à 5 nm.

3. Nanoparticules destinées à une utilisation selon l'une des revendications 1 ou 2, **caractérisées en ce que** lesdites nanoparticules comprennent au moins un ion métallique pour l'imagerie IRM $T_1$.

4. Nanoparticules destinées à une utilisation selon l'une des revendications 1 à 3, **caractérisées en ce que** lesdites nanoparticules ont une relaxivité ($r_1$) par particule allant de 50 à 5000 $mM^{-1}.s^{-1}$ et/ou un rapport massique en ion métallique d'au moins 5%, par exemple allant de 5 à 50%.

5. Nanoparticules destinées à une utilisation selon l'une des revendications 1 à 4, **caractérisées en ce que** l'ion métallique est un ion métallique radioactif, tel qu'un cation d'un ion métallique radioactif $M^{n+}$, n étant un nombre entier allant de 2 à 4.

6. Nanoparticules destinées à une utilisation selon la revendication 5, **caractérisées en ce que** le cation d'un ion métallique radioactif est un lanthanide, de préférence choisi parmi Dy, Lu, Gd, Ho, Eu, Tb, Nd, Er, Yb ou leurs mélanges.

7. Nanoparticules destinées à une utilisation selon la revendication 6, **caractérisées en ce que** l'agent chélatant est choisi parmi les agents complexants des lanthanides, notamment l'acide diéthylène triamine pentaacétique (DTPA), l'acide 1,4,7,10-tétraazacyclododécane-1,4,7,10-tétraacétique (DOTA), l'acide 1,4,7-triazacyclononane-1,4,7 triacétique (NOTA) ou leurs dérivés.

8. Nanoparticules destinées à une utilisation selon l'une des revendications 1 à 7, **caractérisées en ce que** l'ion métallique est $Gd^{3+}$ et l'agent chélatant est l'acide 1,4,7,10-tétraazacyclododécane-1,4,7,10-tétraacétique (DOTA).

9. Nanoparticules destinées à une utilisation selon l'une des revendications 1 à 8, **caractérisées en ce qu'**elles comprennent en outre un agent fonctionnalisant, de préférence choisi parmi (i) un agent fluorescent, (ii) un polyéthylène glycol (PEG) et (iii) une molécule ciblante pour le ciblage des nanoparticules, greffé à la matrice POS ou à l'agent chélatant.

10. Nanoparticules destinées à une utilisation selon l'une des revendications 1 à 9, **caractérisées en ce que** la dysfonction rénale est une fibrose rénale et/ou une insuffisance rénale.

11. Utilisation des nanoparticules telles que définies dans les revendications 1 à 10, comme agent d'imagerie dans le diagnostic *in vivo* d'une dysfonction rénale chez l'homme ou l'animal.

12. Utilisation des nanoparticules telles que définies dans les revendications 1 à 10, pour la préparation d'une composition destinée au diagnostic d'une dysfonction rénale chez l'homme ou l'animal.

13. Méthode pour diagnostiquer une dysfonction rénale *in vivo* chez l'homme ou l'animal auquel ont été administrées des nanoparticules de nanoparticules telles que définies dans les revendications 1 à 10, de préférence comprenant les étapes de :

(i) capture des images par une technique d'imagerie appropriée afin de visualiser au niveau du rein, de préférence au niveau du cortex rénal, lesdites nanoparticules ;
(ii) détermination du rehaussement ;
(iii) comparaison du rehaussement obtenu à l'étape (ii) à un rehaussement de référence.

14. Méthode de suivi de l'efficacité thérapeutique du traitement d'une dysfonction rénale chez l'homme ou l'animal auquel ont été administrées des nanoparticules telles que définies dans les revendications 1 à 10, ladite méthode comprenant les étapes suivantes :

(i) on capture des images par une technique d'imagerie appropriée afin de visualiser au niveau du rein, de préférence au niveau du cortex rénal, lesdites nanoparticules,
(ii) on détermine le rehaussement;
(iii) on répète les étapes (i) et (ii) au cours du traitement du sujet, autant que nécessaire ;
(iv) on déduit l'efficacité thérapeutique du traitement en comparant l'évolution du rehaussement au cours du traitement.

15. Méthode de suivi de l'efficacité thérapeutique d'un traitement d'une dysfonction rénale chez l'homme ou l'animal, ladite méthode comprenant les étapes suivantes:

(i) on administre au patient, des nanoparticules telles que définies dans les revendications 1 à 10, à titre d'agent d'imagerie,
(ii) on capture des images par une technique d'imagerie appropriée afin de visualiser au niveau du rein, de préférence au niveau du cortex rénal, lesdites nanoparticules

(iii) on détermine le rehaussement ;
(iv) on répète les étapes (i) et (iii) au cours du traitement du sujet, autant que nécessaire ;
(v) on déduit l'efficacité thérapeutique du traitement en comparant l'évolution du rehaussement au cours du traitement.

FIGURE 1

FIGURE 2

FIGURE 3

FIGURE 4

FIGURE 5

FIGURE 6

FIGURE 7.

FIGURE 8.

FIGURE 9

Europäisches
Patentamt

European
Patent Office

Office européen
des brevets

# RAPPORT DE RECHERCHE EUROPEENNE

Numéro de la demande

EP 16 30 5038

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| A | LINGZHI HU ET AL: "Assessing intrarenal nonperfusion and vascular leakage in acute kidney injury with multinuclear 1 H/ 19 F MRI and perfluorocarbon nanoparticles", MAGNETIC RESONANCE IN MEDICINE., vol. 71, no. 6, 8 août 2013 (2013-08-08), pages 2186-2196, XP055283566, US ISSN: 0740-3194, DOI: 10.1002/mrm.24851 * abrégé * * page 2187, colonne 2, alinéa 1 * * figure 5 * * page 2195, colonne 1, alinéa 4 * | 1-15 | INV. A61K49/12 A61K49/18 A61K51/06 A61K51/12 B82Y15/00 |
| A | KR 2013 0094875 A (KYUNGPOOK NAT UNIV IND ACAD [KR]) 27 août 2013 (2013-08-27) * alinéa [0074] - alinéa [0109] * | 1-15 | |
| A,D | WO 2011/135101 A2 (NANOH [FR]; UNIV LYON 1 CLAUDE BERNARD [FR]; INST NAT SCIENCES APPLIQ) 3 novembre 2011 (2011-11-03) * exemples 1-20 * | 1-15 | |
| A,D | ANNA MIGNOT ET AL: "A Top-Down Synthesis Route to Ultrasmall Multifunctional Gd-Based Silica Nanoparticles for Theranostic Applications", CHEMISTRY - A EUROPEAN JOURNAL., vol. 19, no. 19, 3 mai 2013 (2013-05-03), pages 6122-6136, XP055283579, WEINHEIM, DE ISSN: 0947-6539, DOI: 10.1002/chem.201203003 * abrégé * * figure 1 * * page 6133, colonne 1, alinéa 2 - page 6134, colonne 1, alinéa 2 * | 1-15 | |

**DOMAINES TECHNIQUES RECHERCHES (IPC)**

A61K

-/--

Le présent rapport a été établi pour toutes les revendications

| Lieu de la recherche | Date d'achèvement de la recherche | Examinateur |
|---|---|---|
| La Haye | 27 juin 2016 | Monami, Amélie |

CATEGORIE DES DOCUMENTS CITES

X : particulièrement pertinent à lui seul
Y : particulièrement pertinent en combinaison avec un
 autre document de la même catégorie
A : arrière-plan technologique
O : divulgation non-écrite
P : document intercalaire

T : théorie ou principe à la base de l'invention
E : document de brevet antérieur, mais publié à la
 date de dépôt ou après cette date
D : cité dans la demande
L : cité pour d'autres raisons

& : membre de la même famille, document correspondant

EPO FORM 1503 03.82 (P04C02)

page 1 de 2

# EP 3 192 530 A1

**RAPPORT DE RECHERCHE EUROPEENNE**

Numéro de la demande

EP 16 30 5038

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| A | CHARLES TRUILLET ET AL: "Ultrasmall particles for Gd-MRI and 68 Ga-PET dual imaging", CONTRAST MEDIA & MOLECULAR IMAGING, vol. 10, no. 4, 5 décembre 2014 (2014-12-05), pages 309-319, XP055283591, GB ISSN: 1555-4309, DOI: 10.1002/cmmi.1633 * abrégé * * scheme 1 * * page 316, colonne 2, alinéa 2 - page 317, colonne 1, alinéa 4 * * page 318, colonne 1, alinéa 3 *<br>----- | 1-15 | |

DOMAINES TECHNIQUES RECHERCHES (IPC)

Le présent rapport a été établi pour toutes les revendications

| Lieu de la recherche | Date d'achèvement de la recherche | Examinateur |
|---|---|---|
| La Haye | 27 juin 2016 | Monami, Amélie |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
& : membre de la même famille, document correspondant

EPO FORM 1503 03.82 (P04C02)

page 2 de 2

19

EP 3 192 530 A1

**ANNEXE AU RAPPORT DE RECHERCHE EUROPEENNE**
**RELATIF A LA DEMANDE DE BREVET EUROPEEN NO.**

EP 16 30 5038

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

27-06-2016

| Document brevet cité au rapport de recherche | Date de publication | Membre(s) de la famille de brevet(s) | Date de publication |
|---|---|---|---|
| KR 20130094875 A | 27-08-2013 | AUCUN | |
| WO 2011135101 A2 | 03-11-2011 | EP 2563408 A2 | 06-03-2013 |
| | | FR 2959502 A1 | 04-11-2011 |
| | | US 2013195766 A1 | 01-08-2013 |
| | | WO 2011135101 A2 | 03-11-2011 |

Pour tout renseignement concernant cette annexe : voir Journal Officiel de l'Office européen des brevets, No.12/82

EPO FORM P0460

20

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2011135101 A

- WO 2013153197 A

**Littérature non-brevet citée dans la description**

- **CORESH J ; BYRD-HOLT D ; ASTOR BC et al.** Chronic kidney disease awareness, prevalence, and trends among U.S. adults, 1999 to 2000. *JAm Soc Nephrol,* 2005, vol. 16, 180-8
- **G.T HERMANSON.** Bioconjugate Techniques. Académie Press, 1996

- Fluorescent and Luminescent Probes for Biological Activity. Académie Press, 1999
- **MIGNOT et al.** A Top-Down Synthesis Route to Ultrasmall Multifunctionnal Gd-Based Silica Nanoparticles for Theranostics Applications. *Chem. Eur. J.,* 2013, vol. 19, 6122-6136